# EUROPEAN PATENT APPLICATION

(11) **EP 2 543 364 A1**
(43) Date of publication of application: **09.01.2013**
(21) Application number: 12187139.6
(22) Date of filing: 14.10.2010
(51) Int. Cl.: A61K 9/20, A61K 31/23, A61K 31/231

(54) **Formulations of cetyl myristate and/or cetyl palmitate**

(62) Divisional of application: 10013657.1
(71) Applicant: Deva Holding Anonim Sirketi, 34303 Istanbul (TR)
(72) Inventor: Haas, Philipp Daniel, 34303 ISTANBUL (TR); Firat, Omer Faruk, 34303 Istanbul (TR); Kandemir, Levent, 34303 ISTANBUL (TR); Ozbek, Mahmut, 34303 ISTANBUL (TR); Koc, Fikret, 34303 ISTANBUL (TR)

(57) **Abstract**

This invention is related to using of anti-adherents in pharmaceutical or dietary supplement formulations of cetyl myristate or cetyl palmitate or combination of cetyl myristate and cetyl palmitate. In this invention, cetyl myristate or cetyl palmitate or combination of cetyl myristate and cetyl palmitate are used as active pharmaceutical ingredients.

## Description

### Technical Field

This invention is related to using of antiadherents in pharmaceutical or dietary supplement formulations of cetyl myristate or cetyl palmitate or combination of cetyl myristate and cetyl palmitate. In this invention, cetyl myristate or cetyl palmitate or combination of cetyl myristate and cetyl palmitate are used as active pharmaceutical ingredients and/or dietary supplements.

### Background Art

Cetyl palmitate is derived from the fatty acid, palmitic acid which occurs as the glycerol ester in many oils and fats such as palm oil or Chinese **vegetable** tallow. A synthetic method of preparation is to react palmitoyl chloride and cetyl alcohol in the presence of magnesium. See the Merck Index, 12th edition at page 336. Reference is also made to **US** US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstruc tive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses using of antiadherents in pharmaceutical or dietary supplement formulations of cetylated fatty acids, especially cetyl myristate or cetyl palmitate or combination of cetyl palmitate and cetyl myristate. Additionally, preparing of them is executed under or equal 45°C environment and/or machines for tabletting or capsulation include a cooling system wherein its temperature does not exceed 45.

### Technical Problem

Cetyl myristate or cetyl palmitate or combination of cetyl myristate and cetyl palmitate can be used as active pharmaceutical ingredients (API) in pharmaceutical formulations in addition to excipient properties. However both cetyl myristate and cetyl palmitate are waxy ingredients and thus in formulation stage, they are adhered onto the surfaces of formulation equipments. It brings about difficulties such as filling of capsules. Therefore it is needed that non-adhering and eligible formulations.

### Solution to Problem

It is invented that sticking problem is solved by using of antiadherents,further excipients and active ingredient(s) with certain percentages in formulations. Using of certain percentages of antiadherent provides to obstacle bedaubing.

### Description of embodiments

Cetyl myristate and cetyl palmitate are sticky and tend to adhere to surfaces of formulation equipments.

This invention discloses processability of cetyl myristate and cetyl palmitate and also the ability not to stick or adhere to equipments.

In accordance with this invention, antiadherents are used in certain percentages to prevent sticking. Antiadherents can be selected from excipients which have only antiadherent function or multifunction wherein at least one function is antiadherent.

Antiadherents are but not limited to talc, colloidal silicon dioxide, calcium carbonate, magnesium trisilicate, calcium stearate,glyceryl behenate, poly(ethylene glycol), magnesium stearate,kaolin ,calcium sulfate, calcium chloride, corn starch,hydrogenated vegetable oil, mineral oil, stearic acid, mixtures thereof and the like.

The anti-adherent excipient or mixtures of excipients can be present in an amount in the range of 0.1 % to 20.0%, preferably 5% to 10%, more preferably 8.3% by weight of pharmaceutical composition. Most preferably anti-adherent excipient or mixtures of excipients are 35 mg of 420 mg in toto formulation.

In accordance with present invention, the pharmaceutical composition of cetyl myristate and cetyl palmitate combination comprises binder,diluent/filler,coating agents and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Diluents/fillers are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst™, mixtures thereof and the like.

Plasticizers are , but not limited to, polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil, acetylated monoglycerides, mixtures thereof and the like. Plasticizer is used to have hard granulate.

According to foregoing explanations fromulation comprises filler is from about 0,5 % to about 60 % , cetyl myrisate as active pharmaceutical ingredient is from about 50 % to about 99,5 % ,cetyl palmitate as active pharmaceutical ingredient is from about 0,5 % to about 10 %, plasticizer is from about 0,1 % to about 6 %, binder is from about 1 % to about 10 % by weight.

Preferably formulation comprises filler is 4,7 % , cetyl myrisate as active pharmaceutical ingredient is 79,3 % ,cetyl palmitate as active pharmaceutical ingredient is 3,9 %, plasticizer is 1,1 %, binder is 2,3 % by weight.

Most preferably, formulation comprises filler is 20 mg , cetyl myrisate as active pharmaceutical ingredient is 333,3 mg ,cetyl palmitate as active pharmaceutical ingredient is 16,7 mg, plasticizer is 5 mg, binder is 10 mg.

In another aspect ,this invention provides a weight ratio of antiadherent or mixtures of antiadherents to total pharmaceutical composition from 1:3 to 1:20. Preferably weight ratio is 1:12.

In another aspect ,this invention provides a weight ratio of antiadherent or mixtures of antiadherents to active ingredient(s) from 1:2 to 1:20. Preferably weight ratio is 1:10.

In yet another aspect, it is invented that pharmaceutical composition of cetyl myristate or cetyl palmitate or combination of cetyl myristate and cetyl palmitate should be pressed as a tablet or should be filled under 45°C. Exceeding 45°C, active ingredient becomes a coagulated and smeared form and pressing or filling is very difficult. As tablet press or capsule filling machine warms itself while working, the condition of 45°C should be maintained in the course of filling or pressing and or tablet press or capsule filling machine contains a cooling system.

### Example

**Table 1**

| **INGREDIENTS** | **mg** |
|---|---|
| Cetyl Myristate/API | 333.3 |
| Cetyl Palmitate/API | 16.7 |
| Diluent/Filler | 20.0 |
| Binder | 10.0 |
| Plasticizer | 5.0 |
| Antiadherent Agents | 35 |
| **TOTAL** | **420.0** |

## Claims

1. A pharmaceutical or dietary composition of cetyl myristate or cetyl palmitate or combination of cetyl myristate and cetyl palmitate **characterized in that** ingredients are filled into capsule and/or pressed as tablet under 45°C environment through using a tablet press or a capsule filling machine which includes a cooling system wherein its temperature does not exceed 45°.
